(19) Europäisches Patentamt / European Patent Office / Office européen des brevets

(11) **EP 2 430 447 B1**

(12) **EUROPEAN PATENT SPECIFICATION**

(45) Date of publication and mention
of the grant of the patent:
**11.05.2016 Bulletin 2016/19**

(51) Int Cl.:
**G01N 33/536** *(2006.01)*    **G01N 33/542** *(2006.01)*
**G01N 33/58** *(2006.01)*    **B82Y 15/00** *(2011.01)*

(21) Application number: **10725219.9**

(22) Date of filing: **30.04.2010**

(86) International application number:
**PCT/FI2010/050354**

(87) International publication number:
**WO 2010/128203 (11.11.2010 Gazette 2010/45)**

(54) **METHOD FOR FINGERPRINTING SAMPLES SUCH AS WINE OR WATER**

VERFAHREN FÜR FINGERPRINTING VON PROBEN WIE WEIN ODER WASSER

MÉTHODE POUR RÉALISER DES EMPREINTES D'ÉCHANTILLONS TELS QUE VIN OU EAU

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **04.05.2009 FI 20095501**

(43) Date of publication of application:
**21.03.2012 Bulletin 2012/12**

(73) Proprietor: **Aqsens Oy**
**02150 Espoo (FI)**

(72) Inventors:
• **HÄNNINEN, Pekka**
**FI-20810 Turku (FI)**
• **HÄRMÄ, Harri**
**FI-20100 Turku (FI)**

(74) Representative: **Berggren Oy Ab**
**P.O. Box 16**
**00101 Helsinki (FI)**

(56) References cited:
**WO-A1-2007/077291    WO-A2-00/16101**
**WO-A2-2004/048937    US-A1- 2009 017 477**

• **NAREOJA T ET AL: "Study on nonspecificity of an immuoassay using Eu-doped polystyrene nanoparticle labels" JOURNAL OF IMMUNOLOGICAL METHODS, ELSEVIER SCIENCE PUBLISHERS B.V.,AMSTERDAM, NL LNKD- DOI:10.1016/J.JIM.2009.04.008, vol. 345, no. 1-2, 18 April 2009 (2009-04-18), pages 80-89, XP026132878 online ISSN: 0022-1759 [retrieved on 2009-04-18]**
• **SEYDACK M: "Nanoparticle labels in immunosensing using optical detection methods" BIOSENSORS AND BIOELECTRONICS, ELSEVIER BV, NL LNKD- DOI:10.1016/J.BIOS.2004.11.003, vol. 20, no. 12, 15 June 2005 (2005-06-15) , pages 2454-2469, XP004861160 ISSN: 0956-5663**
• **GILL I ET AL: "Bioencapsulation within synthetic polymers (Part 1): sol-gel encapsulated biologicals" TRENDS IN BIOTECHNOLOGY, ELSEVIER PUBLICATIONS, CAMBRIDGE, GB LNKD- DOI:10.1016/S0167-7799(00)01457-8, vol. 18, no. 7, 1 July 2000 (2000-07-01), pages 282-296, XP004908534 ISSN: 0167-7799**

**Description**

FIELD OF THE INVENTION

This invention relates to a method for fingerprinting a sample.

BACKGROUND OF THE INVENTION

Binder arrays have been used in detection of biomolecular liquids. The basis of these binder arrays has been the production of a library of components on surfaces or production of e.g. ion sensitive/selective surfaces. When the array is allowed to be in contact with the sample, components from the sample may interact with the array creating a fingerprint that describes the contents of the sample. The array designs vary from specific arrays that detect no or only a few more species that the dimension of the array to non-specific arrays that detect higher number of species. Arrays based on chemical libraries and affinity molecules have been reviewed e.g. in Journal of Biomedicine and Biotechnology; 2003:5 (2003) 257-266. In the field of the invention both electronic interaction based and luminescence/light interaction based detection schemes have been proposed. Whereas non-specific interactions are widely used in sensing odors and volatile compounds (artificial olfaction) only a few examples can be found in detection of compounds from liquid phase.

Specific arrays have widely been applied in proteomics and genomics. In WO/2001/055702 a portable sensor array is disclosed. The array uses microparticles that are in their preferred embodiment depicted in cavities. The publication also suggests various ways of detection scheme including fluorescence quenching and resonance energy transfer. The main idea is to use specific "receptors" as binders on these particles and the publication lists several examples of specific receptors. The publication also reviews the area of sensor array detection.

An array may also be viewed as a multiplex of particles. In a flow fluorometry based detection scheme (Luminex Corporation, www.luminexcorp.com), small (2 to 3 micrometer) microparticles are coded for different categories by internal fluorescent colours. The particles are coated bioactively and the surface attracts both the sample and a fluorescent marker. When the intensity of the fluorescent marker and the internal color coding are read in a flow fluorometric device, quantities of binding for each category in this multiplexed assay become apparent. Similar approaches have been suggested also by others. (US 5,891,738; WO/2003/042698).

In publication by Yuri Vlasov (IUPAC Technical report, Pure Appl.Chem., Vol.77, No.11, pp.1965-1983, 2005) in their wording "non-specific" potentiometric (electronic) sensor arrays and the analysis methods for the results are reviewed. The function of these sensors bases on sensing of ionic currents and typically they operate on micromolar to millimolar concentration range of the ions. The article begins with an explanation of Nikolsky-Eisenman equation that describes the selectivity of a sensor - low selectivity is seen as a problem and is either avoided by better design of the sensing system, or when not possible mathematically using signal processing and statistical analysis. It is clear that these sensors may only detect ionic small compounds such as metal ions. The publication also lists several typical ions that such detectors are tuned for - these ions include ions of iron (Fe), copper (Cu), chlorine (Cl) and sodium (Na). The article also presents (refers to) an analysis of more complex liquids (e.g. wine) in combination with sophisticated signal analysis (Principal component analysis). The described method allowed separation of two different red wines from different areas from Italy and made of a different grape as well as separation of red wines from a white wine.

Non-specific interactions have also been observed between specific capture molecules and molecules that are not their specific counterparts. In WO/2004/048937 an array sensor based on fluorescently labeled oligonuclotides to analyze vapor phase components is presented. The main idea of the technology is to observe changes in the fluorescence properties of the labeled oligonucleotides due to different volatile compounds.

In another approach, the Raise-technology (www.graffinity.com) a well characterized chemical "fragment" library is transferred to an array. These library elements are serving as fragments in lead compound screening of drug discovery studies. Although weak in specificity, the technology aims to find specific interactions between the sample and the array. Detection of these interactions follows by surface plasmon resonance (SPR).

Chemical interactions between specially synthesized chemical compounds (residues) and proteins have been demonstrated by You, Miranda et. al (Nature Nanotechnology, Vol 2., 2007, pp 318-323). The technique bases on synthesis of different chemical residues on metallic nanoparticles. These particles are then allowed to interact with proteins. Depending on the structure of the protein these particles are bound from their residue non-covalently to the proteins. Detection follows by a fluorophore whose fluorescence is quenched by the metal particle in cases when the particles are not bound to sample proteins - when bound, quenching effect is removed and luminescence of the fluorophore is restored. The article reported that by the use of six differently synthesized residues on nanoparticles allowed reliable separation of seven different proteins by the use of the technique in combination with linear discriminant analysis (LDA). Detection sensitivity of the technology is in sub-micromolar range with the best values approaching nanomolar (4 nM for $\beta$-galactosidase). The principle also requires separation of the different liquid binders and reactions are thus performed in separate wells.

WO 2007/077291 and US 2009/017477 disclose a method for determination of concentration, charge unit or size of a substance to be analysed.

Nareoja et al. (Journal of Immunological Methods, vol 345, no. 1-2, pages 80-89) disclose study on nonspecificity of an immuoassay using Eu-doped polystyrene nanoparticle labels.

WO 00/16101 discloses a microsphere-based analytic chemistry system and a method for making the same.

OBJECT AND SUMMARY OF THE INVENTION

One object of the present invention is to provide a method for fingerprinting a sample such as wine or water according to claim 1.

A further object of the present invention is to provide use of the array in such a method according to claim 6.

According to an embodiment of the invention an array of at least two of different interacting surfaces is employed for determining a sample, wherein at least one of the surfaces comprises a non-specific interacting material. The non-specific interacting material advantageously non-specifically interacts with the sample, at least one labelling reactant, or combination of the sample and at least one labelling reactant. In the embodiment the sample and at least one labelling reactant is introduced to said interacting surfaces of said array, such as e.g. letting them in contact with said interacting surfaces of said array. The labelling reactant either as such or in combination with said sample is adapted to change at least one electromagnetically readable property of at least one interacting surface of said array. The electromagnetically readable property may be e.g. optically or radioactively readable property, such as luminance, absorption, emission, reflectivity, polarization, scattering, raman scattering, chemiluminance, radioactive emission, or local change (enhancement) of electromagnetic field. It is to be noted that also at least one further labelling reactant may be employed before obtaining a fingerprint of the sample as is discussed elsewhere in this document.

In order to obtain the fingerprint of the sample the electromagnetically readable property of at least one of said at least two different interacting surfaces of said array is detected at a predetermined time point. The determining the sample is carried out by comparing the fingerprint of the sample with

  i) at least one fingerprint of at least one corresponding sample,
  ii) at least one fingerprint of an array obtained without a sample, and/or
  iii) at least one fingerprint of known samples.

According to an embodiment the sample and/or at least one labelling reactant is introduced to a dilution medium before introducing to the interacting surfaces of said array. Advantageously the dilution medium comprising said sample and/or at least one labelling reactant is introduced to the interacting surfaces of said array so that the sample and/or at least one labelling reactant are not brought directly in contact with the interacting surfaces but via said dilution medium.

According to an embodiment the sample and/or at least one labelling reactant is introduced into a liquid medium before introducing to the interacting surfaces of the array so that the sample and/or at least one labelling reactant are not brought directly in contact with the interacting surfaces but via said liquid medium. It should be noted that even it is disclosed that it is the liquid medium which is introduced to the interacting surfaces, the sample and/or at least one labelling reactant may exist as a gas or solid particles in the liquid.

According to an embodiment the sample may be introduced to the interacting surfaces of said array before any labelling reactant. According to another embodiment at least one labelling reactant may be introduced to the interacting surfaces of said array before the sample, whereafter the sample may interact with the labelling reactant and/or the interacting surface. In addition according to an embodiment the sample and at least one labelling reactant may be introduced to the interacting surfaces of said array together.

According to an embodiment the interacting surfaces are washed with at least one washing medium before detecting said electromagnetically readable property of said interacting surfaces. The interacting surfaces may be washed several times, such as e.g. after introducing first labelling reactant, after introducing the sample and/or after introducing any possible subsequent labelling reactant.

The labelling reactant used in the embodiment of the invention may comprise at least one of the following: luminophore label, radioactive label, and/or a label changing at least luminance, absorption, emission, reflectivity, scattering, raman scattering, chemiluminescence, radioactive emission, local electromagnetic field and/or polarization of at least one interacting surface of the array when introducing said labelling reactant to interact with said interacting surface.

According to an embodiment the luminophore may be selected from the group consisting of coumarins; rhodamines; cyanines; boron-dipyrromethenes; lanthanide compounds, preferably chelates and cryptates; porphyrins; metalloporphyrins; fluorescent proteins; fluorescent polymers; particulate labels, preferably quantum dots, luminescent crystals and luminescent polymer particles; and any combination thereof.

According to an embodiment an essential portion, i.e. at least 10 %, preferably at least 30 %, even more preferably at least 50 % of the interacting surfaces of the array used in the embodiments are selected from groups of interacting surfaces selected from peptides, proteins, detergents, surfactants, carbohydrates and/or derivatives, and polymers. The polymer may be physically treated or chemically treated with an acid, base and/or solvent. For example the pattern of the interacting surface may be treated so its interacting surface area is increased, whereupon the interacting surface may interacting, such as bind, the sample and/or labelling reactants more powerfully and thereby increasing sensitivity.

According to an embodiment the array may comprise at least three interacting surfaces, where the two of which comprise similar type interacting surfaces. When the array comprises similar type interacting surfaces the sensitivity of the array may be enhanced remarkably, especially when the concentration of the sample and/or labelling reactant is minimal, because the probability that at least minor part of the sample and/or labelling reactant would interact with at least one interacting surface is increased.

According to an embodiment the array may comprise plurality of same type interacting surfaces, the interacting force, such as binding force of which is adapted to change gradually or continuously between the minimum and maximum interacting force values. This can be achieved e.g. by changing the thickness, density or concentration of the interacting surfaces. According to an embodiment the array may comprise plurality of interacting surfaces, the type of which is adapted to change gradually or continuously from one type to another type. The array having interacting surfaces with changing interacting property may be used for detecting more fine-grained fingerprints, since much smaller differences with interacting properties of different samples and/or labelling reactants can be detected.

According to an embodiment at least 1, preferably at least 2, even more preferably at least 3 of the interacting surfaces are selected from each of at least 2, preferably at least 3, of the groups of interacting surfaces selected from the peptides, proteins, detergents, surfactants, carbohydrates and/or derivatives, and polymers.

According to an embodiment the array may also comprise an additional surface, such as a gel-like surface, which essentially encapsulates the interacting surfaces. The additional surface is advantageously adapted to convey at least part of said sample, at least one labelling reactant, and/or combination of the sample and at least one labelling reactant through said additional surface in order to interact with said interacting surfaces of the array encapsulated by said additional surface. According to an embodiment the sample can be caught even from the air by the array having additional surface.

Definitions

In this disclosure, the term *array* refers to a particular plurality of different interacting, such as binding surfaces. Accordingly not all interacting surface of the array, i.e. elements of the array, are identical. However, the array can, and in many preferred embodiments does, comprise, in addition to a plurality of different interacting surfaces, also a plurality of each different type of interacting surface.

In the context of the present application the term interacting *surface* or *binding surface* shall be understood to refer to any surface or surfaces that form a uniformly coated or modified electromagnetically (e.g. optically or radioactively) readable and identifiable area with the potential of interacting. Thus each interacting surfaces typically comprises an area of at least 200 nm (nanometer) in diameter and up to 100 mm$^2$ (square millimetres). In many preferred embodiments the diameter is from 0.01 to 10 mm, in more preferred embodiments from 0.5 to 3 mm in diameter. If each interacting surface is that of a particle, the preferred diameter of the particle is from 200 nm to 20 $\mu$m (micrometer) and a more preferred diameter from 1 to 5 $\mu$m. It should be noted that the sample and/or labelling reactant advantageously interact somehow with the interacting sample, such as change the electromagnetically readable property of the interacting surface. According to an embodiment sample and/or labelling reactant may bind e.g. chemically to the interacting surface, whereupon the term binding surface may be used.

It is clear to the skilled in art that the surface area may in some cases be smaller or larger than described above.

The term *analyte* or *analytes* refers, in the context of this application, to any constituent of the sample to be characterized and/or determined, which constituent contributes to the fingerprint of the sample in any specific embodiment of the invention.

The term *plurality* shall be understood to mean more than one, preferably at least three, more preferably at least ten, even more preferably at least 30. It is clear for the person skilled in the art (PSA) that in some preferred embodiments the term plurality can even refer to more than 100, 300, or 1 000.

The term *sample* shall, in the context of this application, be understood to cover any sample to be characterized and/or determined. The sample may be in liquid medium or form, preferably as such, or transformable into liquid form by dissolving it in a dilution medium. The sample typically comprises at least 100 nl but not more than 10 ml. The sample size is preferably from 1 $\mu$l to 1 ml, more preferably from 3 $\mu$l to 300 $\mu$l and most preferably from 10 $\mu$l to 100 $\mu$l.

The term *dilution medium* refers, in the context of this application, to any liquid medium suitable for dilution of the sample in a particular embodiment of the invention. Typically the dilution medium is an aqueous buffer.

The term *luminophore label* refers to a label that comprises a luminophore, i.e. inorganic or organic luminescent matter.

The term *luminophore* refers to an atom, group or particulate, i.e. of a luminophore label, that manifests luminescence and detection of the label accordingly consists of measuring the luminescence of the luminophore. Luminophores comprise e.g. of fluorophores, phosphors, but also conjugated pi systems and transition metal complexes. Luminophores preferred in embodiments of the present invention can be selected from the group consisting of coumarins, rhodamines, cyanines, boron-dipyrromethenes, lanthanide compounds (such as chelates and cryptates), porphyrins, metalloporphyrins, fluorescent proteins, fluorescent polymers, particulate labels (such as quantum dots, luminescent crystals and luminescent polymer particles). Luminophore may also be understood as a complex that is formed of a luminescent molecule or particle as defined above and non-luminescent molecules or molecular complexes.

The term *washing medium or liquid,* be it a *first* or *second* washing liquid, refers according to an embodiment to any liquid applicable for washing the array in any desired step of the method of the present invention. A washing medium or liquids are used in many preferred embodiments of the present invention to enable and/or improve characterization and/or determination. Typically a washing liquid is an aqueous buffer.

The terms *detect* and *detecting* shall be understood, in the context of this invention, to refer to any applicable measurement of luminescence of interacting surfaces of the array. Measurement can be accomplished e.g. using a luminescence plate reader, a dedicated luminescence array reader, a flow luminometric device and/or an automated imaging device.

The term *fingerprint* refers, in the context of the present application, to the array of results obtained through detection, i.e. measurement, of the plurality of different interacting surfaces of the array measured in any embodiment of the invention. If an embodiment of the present invention involves also a plurality of identical interacting surfaces and more than one of these are detected, the fingerprint can comprise all the results of the identical interacting surfaces or alternatively only a representative value, e.g. average, median, mode of the measurements of identical interacting surfaces or any combination thereof [Bartz A.E. (1999), Basic Statistical Concepts. 4th ed., Upper Saddle River, NJ: Prentice Hall.]. A fingerprint can further refer to a profile of measured luminescent intensities subjected to numerical processing with an appropriate algorithm and in many preferred alternatives measured luminescent intensities of the interacting surfaces of the array are subjected to numerical processing by an appropriate algorithm before comparison with fingerprints of corresponding arrays without a sample and/or known samples.

The term *corresponding sample* or *corresponding samples* refer to samples that are believed to be virtually identical, highly similar or at least reasonably similar to those characterized and/or determined. This belief of similarity can be due to e.g. origin, i.e. relating to the same or a corresponding process or product, or classification.

The term *known samples* refers to any samples which composition is known in detail or is fully characterized.

The terms *non-specific* interacting means or *non-specific binder* refers e.g. to a binder or binders that are, in the context of a specific embodiment of the present invention, not specific: the selectivity of the binding is not predetermined. Accordingly a binder, which in some other context is a specific binder, might, in the context of the present invention be a non-specific binder due to that binding in the context of the present invention is not specific. Preferably the non-specific binder or binders of the present invention are not specific binders in any context.

The terms *specific* interacting means or *specific binder* refers e.g. to a binder that is specific or binders that are specific. A specific binder comprises a molecular recognition element or elements that bind to an epitope of the analyte bound. In the context of this invention a specific binder only binds to similar epitopes, i.e. epitopes that are chemically and structurally similar. A specific binder does not bind to more than 10, preferably not more than 3, chemically and conformationally non-identical different epitopes. Most preferably a specific binder only binds to one specific epitope.

In the contest of this invention the term *bind* refers to binding wherein the binding constant is at least $10^3$ M$^{-1}$, preferably at least $10^5$ M$^{-1}$, more preferably at least $10^7$ M$^{-1}$ and most preferably at least $10^9$ M$^{-1}$.

BRIEF DESCRIPTION OF THE DRAWINGS

Next the invention will be described in greater detail with reference to exemplary embodiments in accordance with the accompanying drawings, in which:

Figure 1 illustrates a principle of an exemplary method for determining a sample according to an advantageous embodiment of the invention.

Figure 2A illustrates an exemplary (one dimensional) array to be used for determining a sample according to an advantageous embodiment of the invention.

Figure 2B illustrates another exemplary array to be used for determining a sample according to an advantageous embodiment of the invention.

Figure 2C illustrates an exemplary (two dimensional) array to be used for determining a sample according to an advantageous embodiment of the invention.

Figure 2D illustrates another exemplary (two dimensional) array to be used for determining a sample according to an advantageous embodiment of the invention.

Figure 2E illustrates exemplary multidimensional array to be used for determining a sample according to an advantageous embodiment of the invention.

Figure 2F illustrates another exemplary array with an additional surface to be used for determining a sample according to an advantageous embodiment of the invention.

Figure 3A illustrates an exemplary arrangement for determining a sample employing an array of at least two of different interacting surfaces according to an advantageous embodiment of the invention.

Figure 3B illustrates exemplary intensities of triggering and emission curves measured by the arrangement according to an advantageous embodiment of the invention.

Figure 4 illustrates exemplary measurement plots of most significant, i.e. principal, components of bottled waters determined according to an advantageous embodiment of the invention.

DETAILED DESCRIPTION OF THE INVENTION

Preferred embodiments of the invention

An exemplary embodiment of the method of the invention for determining a sample employing an array of a plurality of different binding surfaces comprises e.g. the following steps:

a) bringing, in liquid form, a sample either as such, or appropriately diluted in a dilution medium, optionally comprising at least one labelling reactant with a luminophore label, in contact with said array;
b) letting said sample react with said binding surfaces of said array;
c) optionally washing said binding surfaces with a first washing liquid;
d)

i) bringing at least one labelling reactant with a luminophore label in contact with said binding surfaces of said array if said dilution medium comprising said labelling reactant with said luminophore label is not employed in step a); or
ii) optionally bringing at least one further labelling reactant with a luminophore label in contact with said binding surfaces of said array in case said dilution medium comprising said labelling reactant with said luminophore label is employed in step a);

e) optionally washing said binding surfaces of said array with a second washing liquid;
f) detecting at a predetermined time point or time points said luminophore or luminophores on an essential portion, i.e. at least 10 %, preferably at least 30 %, more preferably at least 50 %, even more preferably at least 70 % and most preferably at least 90 %, of said plurality of different binding surfaces of said array to obtain a fingerprint of said sample; and
g) determining said sample by direct comparison, and/or comparison by employing an appropriate algorithm or algorithms for comparison of fingerprint of said sample with

i) a fingerprint of a corresponding sample or fingerprints of corresponding samples,
ii) a fingerprint or fingerprints of an array obtained without a sample, and/or
iii) a fingerprint or fingerprints of known samples;

wherein an essential portion, i.e. at least 10 %, preferably at least 30 %, more preferably at least 50 %, even more preferably at least 70 % and most preferably at least 90 %, of said plurality of different binding surfaces consist of non-specific binders.
In some exemplary embodiments of the invention at least one labelling reactant e.g. with a luminophore label is employed and said labelling reactant binds in the conditions prevailing in the method non-specifically to an essential portion, i.e. at least 10 %, preferably at least 30 %, more preferably at least 50 %, even more preferably at least 70 % and most preferably at least 90 %, of said plurality of different binding surfaces of the array in the absence of a sample. In some of these exemplary embodiments at least one further labelling reactant e.g. with a luminophore label may also be employed and said labelling reactant binds in the conditions prevailing in the method specifically to at least one binding surface of the plurality of different binding surfaces of the array.

In some preferred embodiments of the invention at least 3, preferably at least 5 and most preferably at least 10 different binding surfaces are comprised in the array.

Often the array comprises a plurality of at least one type of binding surface of the different binding surfaces of the array. Typically the array comprises at least 3, preferably at least 5 and most preferably at least 10 of at least 1 preferably 3, even more preferably 10 and most preferably all different binding surfaces comprised in the array.

In exemplary embodiments of the invention an essential portion, i.e. at least 10 %, preferably at least 30 %, even more preferably at least 50 % of the binding surfaces are selected from groups of binding surfaces selected from peptides, proteins, detergents, surfactants and polymers. Preferably at least one binding surface is a polymer, optionally treated with an acid, base and/or solvent.

In exemplary embodiments of the invention at least 1, preferably at least 2, even more preferably at least 3 of the binding surfaces are selected from each of at least 2, preferably at least 3, of the groups of binding surfaces selected from the peptides, proteins, detergents, surfactants and polymers.

Preferably the luminophore or luminophores employed in the invention are selected from the group consisting of coumarins; rhodamines; cyanines; boron-dipyrromethenes; lanthanide compounds, preferably chelates and cryptates; porphyrins; metalloporphyrins; fluorescent proteins; fluorescent polymers; particulate labels, preferably quantum dots, luminescent crystals and luminescent polymer particles; and any combination thereof.

In an exemplary array of a plurality of different binding surfaces an essential portion, i.e. at least 10 %, preferably at least 30 %, more preferably at least 50 %, even more preferably at least 70 % and most preferably at least 90 %, of said plurality of different binding surfaces consist of non-specific binders.

In exemplary embodiments at least 3, preferably at least 5, more preferably at least 10 and most preferably at least 30 different binding surfaces are comprised in the array.

In exemplary arrays an essential portion, i.e. at least 10%, preferably at least 30 %, even more preferably at least 50 % of the binding surfaces are selected from groups of binding surfaces selected from peptides, proteins, detergents, surfactants and polymers. Preferably at least one binding surface is a polymer, optionally treated with an acid, base and/or solvent.

In exemplary embodiments of the array at least 1, preferably at least 2, even more preferably at least 3 of the binding surfaces are selected from each of at least 2, preferably at least 3, of the groups of binding surfaces selected from the peptides, proteins, detergents, surfactants and polymers.

The array is typically used for characterizing and/or determining a sample.

According to an exemplary embodiment at least one non-specific label and an array of plurality of different binding surfaces are used, wherein

a) said non-specific label or labels comprise e.g. a luminophore or luminophores are employed;
b) an essential portion, i.e. at least 10 %, preferably at least 30 %, more preferably at least 50 %, even more preferably at least 70 % and most preferably at least 90 %, of said plurality of different binding surfaces consist of non-specific binders;
c) the non-specific label or labels bind non-specifically to said array;
d) a sample to be characterized and/or determined interacts with binding of said non-specific label to said array; and
e) detecting at a predetermined time point or time points said luminophore or luminophores on an essential portion, i.e. at least 10 %, preferably at least 30 %, more preferably at least 50 %, even more preferably at least 70 % and most preferably at least 90 %, of said plurality of different binding surfaces of said array to obtain a fingerprint of said sample is carried out resulting in a fingerprint;

for determining said sample.

One of the basic paradigms of measurement of biomolecular binding reactions has been the specificity of the binder molecules. The higher the specificity, the better the assay. Non-specific binding has without an exception been seen as a burden that limits the sensitivity of the measurement by introducing a background component of often unknown dimension and origin. At least some of the embodiments of the present invention are based on an inverted approach to this traditional specific detection scheme and it discloses how non-specific interaction, such as binding can be used in measurement and analysis e.g. of complex fluids or other samples.

An exemplary method bases on the use of a simple array of non-specific binders, non-specifically reacting luminescent labels and analysis of the sample specific fingerprint on the array. The advantage of using non-specifically reacting components is the fact that a single interacting means, such as binder can interact or bind several different species. The combination of the results from different non-specific interacting means or binders creates a sample specific fingerprint. With a well-designed array the fingerprint as such or the numerically processed fingerprint may be extremely specific and thus a relatively small array of only a few binders can be used in detection of several sample species whereas the dimension of the array would limit a specific array. A further advantage of the method is the use of direct observation of electromagnetically readable property, such as luminescence intensity as such. From literature it is known that with specific binders and optimal conditions, direct luminescence detection (time-resolved) may reveal concentrations as low as a few attomoles/liter. Even with reduced affinity of binding, the sensitivity of a direct luminescence based method can thus be remarkable. When the exemplary method of this invention is compared with traditional chromatography or mass-spectrometry measurements, it is simple and straightforward. As compared with different electrical sensors or known luminescent sensors, the method offers much higher sensitivity and selectivity due to a higher number of surface coating and modification options offering a larger application scale than the known array screening techniques. When compared with known array sensors based on luminescence and specific interactions the invention offers simplified production and a much wider application area for a single array.

Specific binding in this context is understood as a strong, predetermined and selected binding force between a binder molecule and a single or a limited set of target molecules. Such specific binding may be for example binding between an antibody and its' specific antigen. Non-specific binding, on the contrary, is understood as a binding force of often-undetermined origin that can vary from weak to extremely strong but has little selectivity with the target molecules. Such binding may be for example binding of the sample molecules to the walls of the assay vessel (test-tube). Thus non-specific binders show affinity towards more molecule classes than specific binders, usually 10 or more non-analogous and different targets bind to non-specific binder whereas specific binder typically bind only a single species and its structural analogues.

Specific binding mechanisms are usually well characterized, whereas non-specific binding may take place for a vast number of reasons from simple electrostatic attractive reasons to complicated binding including conformational changes of the binding partners. Binders for a non-specific array include but are not limited to proteins such as albumins; aminoacids; peptides; detergents; surfactants; sugars, such as glucose; glycine; polymers, such as polyethylene glycol, polypropylene glycol, polypropylene, pylyethene, polystyrene and their variants; crafted polymers, functionalized polymers; polyelectrolytes; small molecules; nucleic acids; cells; organisms; tissue; organic and inorganic materials; metals; crystalline or amorphous materials; ionic and non-ionic compounds. Small molecules refer, in this context, to molecules with a molecular weight (MW) of less than 10 000, preferably less than 1 000. Referral to cells can refer to wild type cells or genetically modified cells. The cells can be animal, including human, cells, bacteria or viruses. Binders may also introduce chirality to the surface. These binders can also be used as a mixture and their derivatives. A non-specific binder can also be a chemically or structurally modified surface, a hydrophobic or hydrophilic surface or the surface may contain halide, sulphate, carboxy, amine, thiol, hydroxyl, ketone, ether, epoxide, aldehyde and organometallic groups. The surface may also be treated with e.g. acids, bases or solvents.

Contrary to non-specific binding reactions specific binding reactions are characterized e.g. either as receptor-ligand or ligand-fragment interactions or nucleic acid hybridization reactions. Specific binding reactions in this context are typically also all reactions that trigger or catalyze an event or events, e.g. protein interactions such as RNA DNA transcription, polymerization, cell signaling and chaperon assisted protein folding.

Because of the vast number of different non-specific interactions, manufacturing of different non-specific binding surfaces is straightforward. In this context "surface" can be that of a microtiter plate well, a spotting slide, a particle or any other surface that forms a uniformly coated or modified optically readable identifiable area of 200 nanometers in diameter to 100 mm$^2$ but typically 0.01 mm to 10 mm and preferably 0.5 mm to 3 mm in diameter. In that the surface forms a particle the preferred diameter of the particle is from 200 nm to 10 micrometers. In some embodiments of the invention the diameter of the particle or surface may also be smaller than 200 nm. A surface may, for example, be modified by charge and nanostructure, or it may be coated by layers of different molecular species and mixtures thereof.

A particle array may be realized in several ways that can be found in literature. In a preferred embodiment of a non-specific particle array the array is read by a flow-fluorometric device (see e.g. www.luminexcorp.com referred to in more detail above). Several other options for reading such an array may also be found in prior art (e.g. WO/2001/055702).

The approach of the present invention offers practically an unlimited non-specific chemical diversity for the surfaces. Specific binders, on contrary, are difficult to produce - for example production of a new antibody is a complex process and requires either a) use of animals or b) large artificial binder library searches. Further production of small molecular binder species, e.g. for drug discovery, requires complex chemical syntheses and a-priori knowledge of binding properties of different molecular groups or residues.

Specific binders may also be used as non-specific binders in cases where the binding partners bind to them without the specific targeted interaction. In a preferred embodiment of the current invention the binding array may also contain specific binders that act both as non-specific and as specific binders but typically less than 50 % of all binders and preferably less than 10 % of the binders of the array.

According to an exemplary embodiment of the present invention, the detection of a sample takes place by using a matrix of carefully selected surface modifications including modifications to the carrier surface nanostructure and charge as well as deposition of molecular layers on the surface. In preferred embodiments of the invention the array is brought into contact with the sample and non-specific interactions of the array and the sample are revealed by a luminophore interacting non-specifically with the array, in particular, with changes (modulation) of intensity of the luminophore, when compared to a reference array, e.g. array of a corresponding sample, array obtained without sample and/or array of known samples. In some embodiments of the invention the modulation may also be observed in the luminescence lifetime, luminescence polarization or luminescence energy transfer. The modulation in luminescence may also be observed as a function of time. Accordingly the invention can also be used for kinetic characterization and/or determining of a sample. In such cases detection of a sample or parallel samples at different time points can be carried out from the same array or from parallel arrays respectively.

Luminophores or components of a mixture of luminophores can be selected from the group consisting of coumarin; rhodamines; cyanines; boron-dipyrromethenes; lanthanide compounds, such as chelates and cryptates; porphyrins; metalloporphyrins; fluorescent proteins; nanolabels, such as quantum dots and other luminescent nanoparticles; luminescent nanocrystals and luminescent polymers.

In some embodiments of the invention the luminophore may consist of a donor and acceptor luminophore or a donor and a quencher such that the acceptor or quencher molecule are brought into contact with the donor luminophore or separated from the donor luminophore by the non-specific reactions of the invention.

In some embodiments of the invention the luminophore may be substituted by molecules or particles of the sample interacting with the array in which embodiments these molecules or particles are distinguishable by e.g. their absorption, scattering, raman scattering, chemiluminescence, radioactive emission and local enhancement of electromagnetic field or via other electromagnetically readable properties discussed e.g. in this document.

In many preferred embodiments of the invention the non-specific array may also contain a reference luminophore that is either a) not participating in the non-specific binding reactions or b) is participating in the reaction in a known fashion. The signal from the reference luminophore may e.g. be used for normalization of the results.

The mechanisms of non-specific interaction, such as binding are numerous, often undetermined and have low specificity and selectivity. The selectivity and specificity of the array depend from the interpretation of the fingerprint of several spots. In a welldesigned array this fingerprint is unique for each sample. In preferred embodiments of the invention the fingerprint is compared to a reference fingerprint or fingerprints. Detection may be carried out e.g. by a luminophore or a mixture of luminophores that also are non-specific binders. In some embodiments of this invention the luminophores may also be specific binders or a mixture of specific and non-specific binders. The luminophore may be used in revealing the fingerprint of an unknown sample by comparing the fingerprint of the luminophore in presence and absence of the sample. In comparative studies the fingerprint of the array is compared in presence of samples A, B, C etc. The comparisons of the acquired fingerprints against a library or comparison sets are made by known methods from bioinformatics and data mining. In many embodiments of the present invention fingerprints processed using appropriate algorithms are compared rather than observing the fingerprints of samples as such. Fingerprints and fingerprints processed using appropriate algorithms of the sample can be compared with fingerprints or fingerprints processed, respectively, using appropriate algorithms of:

1) a sample from the same process taken at an earlier time point,
2) a sample taken before adding a substance to the process under study,
3) a fingerprint of the array in absence of the sample,
4) a library of fingerprints from known samples,
5) a library of fingerprints related to known anomalities observed at the time points of the library sample,
6) an algorithm trained with fingerprints of known samples,
7) an algorithm trained with fingerprints from samples related to known anomalities observed at the time point of the sampling, and or
8) a fingerprint or set of fingerprints recorded as a function of time.

These algorithms [see e.g. www.dtreg.com and Romesburg C, Cluster Analysis for Researchers, Lulu Press 2004] may e.g. be based on:

1) neural networks,
2) independent/principal component analysis,
3) discriminant analysis,
4) other clustering algorithms, and
5) generic expression programming.

In exemplary embodiments of the invention a non-specific luminophore may bind directly to the non-specific array surface. In this case the sample can either increase or decrease the binding of the luminophore onto the surface. In further embodiments the luminophore may also be bound to sample molecules in the solution and thus be prevented from binding to the non-specific surface or be linked to the surface by the sample molecules. Because of these different alternatives modulation of the luminescence of a specific binding surface can either result in increase or decrease of the luminescence but does not necessarily affect the luminescence of a each particular binding surface of the array at all.

In some preferred embodiments of the invention the sample is first diluted in a buffer before contacting the array. The buffer may contain components that enhance modulation of the non-specific binding. These components may act with the sample non-specifically or specifically. The components may contain standard features for the fingerprint that can be used in normalization and interpretation of the results. These components may also act with the array surfaces increasing the probability of the luminophore to be bound or displaced by the sample. The buffer may also contain luminophores for reference purposes. The buffer may also contain the luminophore or luminophores that are used to detect the interaction of the sample and the array. The buffer may also be completely inert and act only as dilutant for the sample. In a preferred embodiment of the invention the pH of the buffer is often < 7, preferably < 5 and typically between 3 and 4 to increase the probability of non-specific binding. The electrolyte concentration of the buffer is typically < 100 mM and preferably < 10mM.

In an exemplary embodiment of the invention the assay is performed as follows.

1) A sample is obtained and preferably but not necessarily diluted in a standard buffer solution.

   a. A standard buffer solution can be used to stabilize the sample or to enhance modulation of the luminescence of the luminophore bound to the array. The solution may also contain substances that result in standard features to determination results of the array to help normalize and interpret the fingerprint.

2) The sample and optionally the standard buffer solution is allowed to react with the non-specific array. If a standard buffer solution is used it may also be in contact with the array before addition of the sample.

3) The array is optionally washed. If it is washed the washing solution may be selected such that it enhances the modulation of the fingerprint. Antioxidants, urea, acids, bases and/or detergents can be used as components of the washing solution to enhance modulation.

4) The luminophore or luminophore mixture is added and allowed to react with the array surface. The luminophore or luminophores may alternatively or additionally be a part of the 1) standard buffer solution if used and may also be in contact with the array before the sample is added.

5) The array is optionally washed as in 3).

6) The arrays are read. Reading may include addition of an enhancement solution that increases, decreases and/or stabilizes the luminescence of the luminophore.

   a. Reading of results may be carried out with e.g.

      i. a luminescence plate reader,
      ii. a dedicated luminescence array reader,
      iii. a flow luminometric device, and/or
      iv. an automated imaging device.

7) The results are interpreted by an appropriate method

   a. The method may involve e.g.

      i. comparison to a sample from the same process taken at an earlier time point,
      ii. comparison to a sample taken before adding a particular substance to the process under study,
      iii. comparison to a fingerprint of the array in absence of the sample,
      iv. comparison to a library of fingerprints from known samples, and/or
      v. comparison to a library of fingerprints related to known anomalies observed at the time points of the library sample

   b. The method may utilize e.g.

      i. an algorithm sensitive in differentiation of multidimensional signals,
      ii. an algorithm trained with fingerprints of known library samples,
      iii. an algorithm trained with fingerprints from known library samples related to known anomalities observed at the time point of the sampling, and/or

iv. comparison to results from the same sample as a function of time after the addition of reaction components.

c. Algorithms of the method may be based on e.g. neural networks, independent component analysis, discriminant analysis and other clustering algorithms and generic expression programming.

It is clear for a person skilled in the art that the presented protocol and the order of the addition of the reaction components may vary depending e.g. on the application, sample, used reaction, washing buffers and used analysis methods. In addition is should be noted that even the luminiphore label is disclosed above, also other label e.g. disclosed in this document can be used for interacting with the interacting surfaces of the array and thereby changing the electromagnetically readable property.

In some embodiments of the invention for detection of water based fluids the array is preferably selected such that it contains non-specific surfaces that are coated with at least one surface from each of the groups: proteins, detergents, polymers and at least one surface that is treated with acids, bases or solvents. In such an array the luminophore is selected such that it is preferably hydrophobic and fluorescent or shows delayed fluorescence or phosphorescence upon excitation with suitable wavelength light. The luminophore may also be a combination or a mixture of a fluorescent substance and a substance exhibiting delayed luminescence upon excitation with suitable wavelength light.

The advantages of the current invention to known methods utilizing non-specificity and array detection are:

1) The method is not limited by the size of the molecules to be detected to small or large molecular compounds but the array can at the same time detect small molecular substances and large molecules and molecule complexes and even organisms.

2) The array may be built in a way that it contains both specific interacting and non-specifically interacting surfaces. In the preferred embodiment of the invention most of the interacting surfaces are non-specific.

3) The method of the invention can be applied using labels selected according to the needs of each specific application. Accordingly labels such as radioactive labels, scattering labels could be applied. Labels may also be used as a mixture.

4) The method according to this invention allows washing of the array surface so that the problems of single step assay formats can be avoided when needed.

5) Especially for larger molecules the method enables a sensitivity that is comparable to any immunoassay method and is thus 3-6 orders of magnitude better than any known chemical or electronic arrays employing non-specificity.

EXAMPLES

**Example 1**

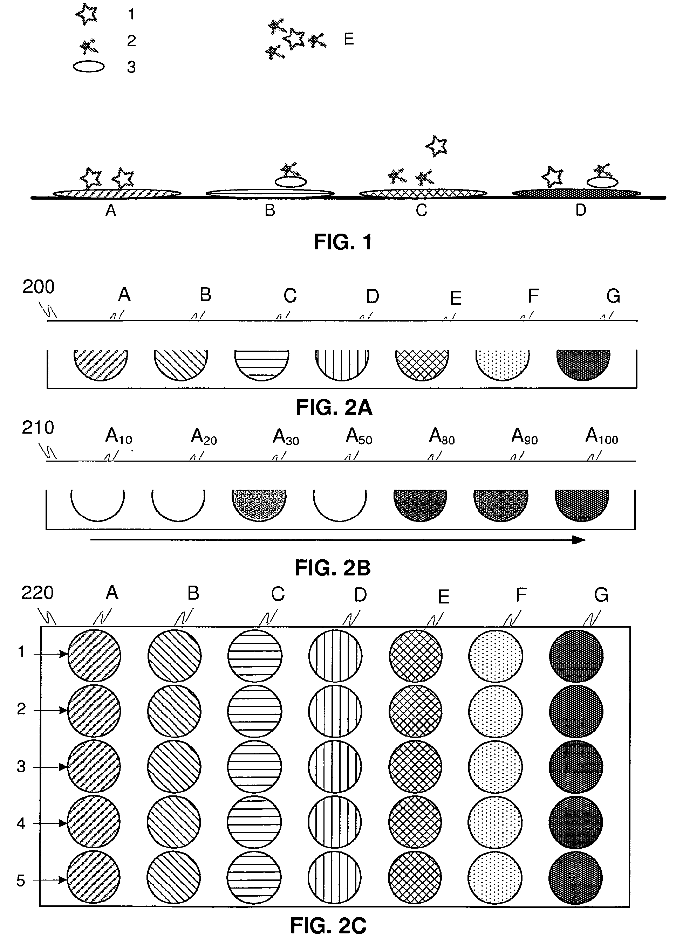

Figure 1 illustrates a principle of an exemplary method for determining a sample employing an array of at least two of different interacting surfaces according to an advantageous embodiment of the invention. The surface spots marked with A, B, C, D represent differently coated or modified surfaces each forming a differently behaving non-specific interacting surface. The sample molecules (2, 3) may interact with the array spots in different ways. In A the sample molecules do not react with the array spots and only the label (1) will bind to the surface. In B the sample molecule reacts with both label (1) and surface bringing the label (1) in contact with the array spot. In this case the label (1) does not bind to the non-specific array component B. In C the sample molecules (2) prevent the binding of the label (1) to the surface. In D both sample molecules (2,3) and label bind to the surface. In E the sample molecules (2) bind to the label molecule (1) inhibiting the molecule to interact with any of the surface spots (A,B,C,D).

In this example each of the surfaces has different type of the electromagnetically readable properties due to different interactions with the samples and/or label, whereupon a specific fingerprint can be determined for each case.

Figure 2A illustrates an exemplary one dimensional array 200 to be used for determining a sample according to an advantageous embodiment of the invention. The exemplary array 200 comprises 7 different interacting surfaces A-G advantageously comprising different interacting surface material, where at least part is non-specific surface material, whereas some of the interacting surface material may be specific surface material, as discussed elsewhere in this document. According to an embodiment the surfaces A-G with different interacting properties may be achieved also by treating physically and/or chemically e.g. so that the interacting abilities such as pattern, surface area or binding properties of each surface differs from each other.

Figure 2B illustrates another exemplary array 210 to be used for determining a sample according to an advantageous embodiment of the invention, where the interacting material of the different interacting surfaces may be the same, such as non-specific interacting material, but the interacting ability, such as binding force, may be different for different surface. In the array 210 the variation of the binding force of the surfaces may be achieved e.g. by treating the surfaces physically and/or chemically. For example the pattern of the interacting surface may be treated so that its interacting surface area is increased such as in Figure 2B, where the interacting force increases in the direction of the arrow. The variation in interacting force may also be achieved for example varying the concentration or density of the interacting material applied in the array. For example the most left interacting surface ($A_{10}$) may be treated so that the concentration of the non-specific surface material (or alternatively its surface area or other property influencing the interacting ability, such as binding force) is only 10% of the maximum ($A_{100}$), the second one ($A_{20}$) has 20%, the third one ($A_{30}$) has 30%, the fourth one ($A_{50}$) has 40%, the fifth one ($A_{80}$) has 80%, the sixth one ($A_{90}$) has 90% of the maximum ($A_{100}$) and the seventh one ($A_{100}$) has the maximum concentration and/or surface area. By the embodiment of figure 2B the more specific array 210 can be achieved since the sample and/or labelling reactant may interact differently e.g. with the interacting surfaces having e.g. different binding forces.

Figure 2C illustrates an exemplary (two dimensional) array 220 to be used for determining a sample according to an advantageous embodiment of the invention, where the array comprises plurality of different kinds of interacting surfaces A-G having different interacting material type and thereby different interacting ability. In addition the array 220 comprises multiple same type interacting surfaces with similar or same interacting ability, such as same interacting material. For example the array 220 comprises five interacting surfaces of different type A-G (A1, A2, A3...). This is advantageous when e.g. the concentration or quantity of the sample to be determined is very minimal, whereupon the probability that at least minor part of the sample and/or labelling reactant would interact with at least one interacting surface is increased remarkably. For example if the sample interacts in theory with the interacting surface type of A, it may happen that there are no interaction with surfaces A1, A2, and A5, but only with surfaces A2 and A4 due to small concentration.

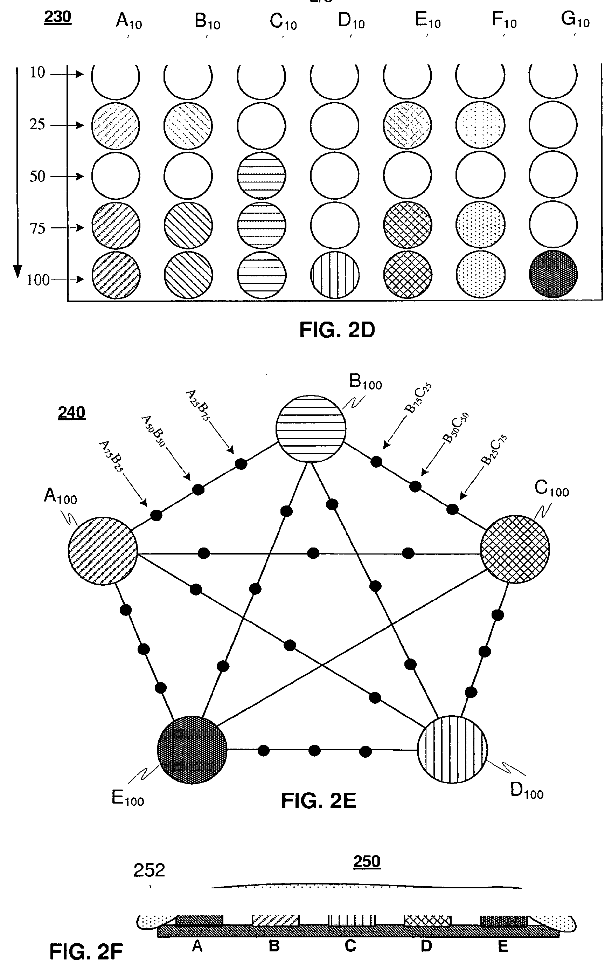

Figure 2D illustrates another exemplary (two dimensional) array 230 to be used for determining a sample according to an advantageous embodiment of the invention, which is as a combination of arrays 200, 210 disclosed in figures 2A and 2B so that it 230 comprises plurality of different interacting surface types A-G, but also plurality of same interacting surface type, such as A, with different interacting force, such as $A_{10}$-$A_{100}$. By the embodiment of figure 2D even more specific array 230 can be achieved since the sample and/or labelling reactant may interact differently e.g. with the interacting surfaces having e.g. different binding forces ($A_{10}$, $A_{25}$, $A_{50}$,...) as well as also as also at the same time with different interacting surface types (A, B, C,...).

Figure 2E illustrates an exemplary multidimensional array 240 to be used for determining a sample according to an advantageous embodiment of the invention, where each of the interacting surface type A-E has at least one interacting surface comprising essentially only interacting surface material characteristic for the type, such as interacting surface $A_{100}$ comprises essentially 100% interacting surface material A, interacting surface $B_{100}$ comprises essentially 100% interacting surface material B, etc. However, in addition the array 240 advantageously comprises plurality of interacting surfaces the interacting surface material of which is mixed at least with one another interacting surface material, such as the interacting surface $A_{75}B_{25}$ which comprises 75% interacting surface material A and 25% interacting surface material B.

Figure 2F illustrates another exemplary array 250 to be used for determining a sample according to an advantageous embodiment of the invention, where array comprises an additional surface 252, such as a gel-like surface, which essentially encapsulates the interacting surfaces. The additional surface is advantageously adapted to convey at least part of said sample, at least one labelling reactant, and/or combination of the sample and at least one labelling reactant through said additional surface 252 in order to interact with said interacting surfaces of the array 250 encapsulated by said additional surface.

It should be noted that the locations of the interface surfaces may differ from the locations of the exemplary arrays 200-250 disclosed in figures 2A-2F.

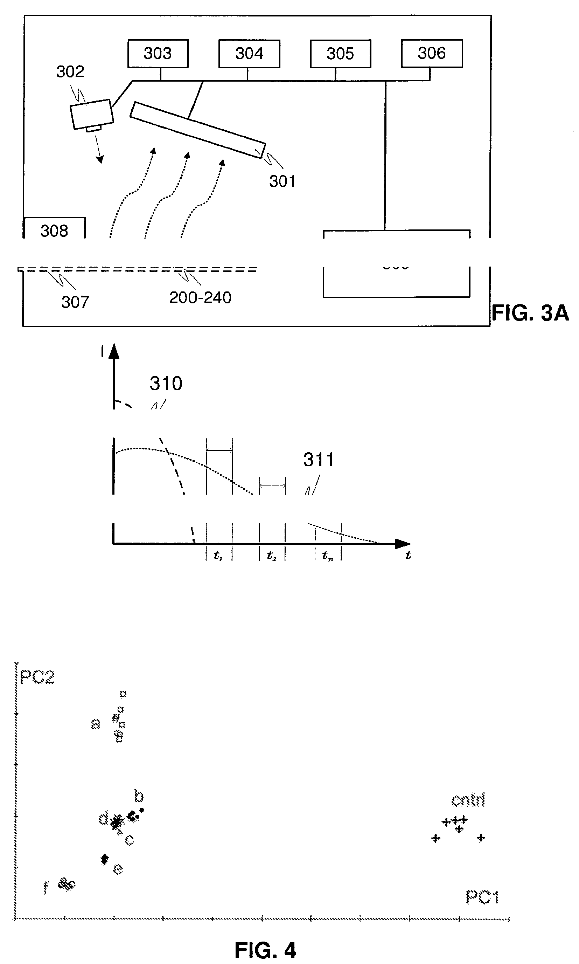

Figure 3A illustrates an exemplary arrangement 300 for determining a sample employing an array 200-240 of at least two of different interacting surfaces according to an advantageous embodiment of the invention, and Figure 3B illustrates exemplary intensities of triggering and emission curves measured by the arrangement 300. The arrangement advantageously comprises at least detecting means 301 for detecting at least one electromagnetically readable property of at least one interacting surface of the array. The detecting means 301 may be for example sensitive for luminescence radiation radiated by the luminophore labels bound by the interacting surfaces of the array, whereupon the fingerprint of the sample can be determined by detecting the intensity of the luminescence of each interacting surface of the array introduced to the sample to be detected and/or characterized.

According to an embodiment the detecting means 301 may be adapted to detect radioactive radiation emitted by the interacting surfaces, especially if a radioactive label is used. However, it is to be noted that also detecting means 301 capable of detecting radiation of another kind can be applied as well.

According to an embodiment the arrangement 300 may also comprise an emitting means 302 for emitting a certain electromagnetic radiation, which reflection, absorption, polarization or scattering, for example, is detected advantageously by the detecting means 301.

In addition, according to an embodiment, the emitting means 302 may be adapted to emit a triggering pulse 310 for triggering e.g. luminescence effect 311 of the luminophore labels interacted with the surfaces of the array. The arrangement advantageously comprises also timing means 303 for triggering the emitting means 302 to emit the radiation, but also triggering the detecting means 301 to detect the electromagnetically readable property, such as luminescence, at an appropriate time point ($t_1$, $t_2$,..., tn) so that the triggering pulse 310 does not disturb the detection of the luminescence radiation 311.

The arrangement may also comprise controlling means 304 for controlling the emitting means 302 as well as the detecting means 301. For example, according to an embodiment, electromagnetically readable property of each individual interacting surface can be detected separately and independently of other surfaces by the detecting means, and/or the emitting means may be adapted to emit e.g. electromagnetic radiation or triggering pulse individually for each individual interacting surface. The controlling means 304 and timing means 303 may be adapted to control the emitting means and detecting means so that the electromagnetically readable property of each interacting surface can be read separately and independently of each other.

The arrangement 300 enables also the determination of kinematic of the emission, such as luminescence lifetime, which may be additional parameter when determining the fingerprint of the sample, since the lifetime may differ for different label reactants or samples.

In addition the arrangement advantageously also comprises comparing means 305 for comparing said fingerprint of said sample with at least one of the following information advantageously stored in memory means 306

    i) at least one fingerprint of at least one corresponding sample,
    ii) at least one fingerprint of an array obtained without a sample, and/or
    iii) at least one fingerprint of known samples,

in order to characterize and/or determine said sample.

Moreover the arrangement may also comprise receiving means 307 for receiving the array 200-240 and washing 308 means for washing the interacting surfaces of the array with at least one washing medium before detecting said electromagnetically readable property of said interacting surfaces. Furthermore the arrangement may also comprise user interface means 309, such as a keyboard, display or other controlling means for inputting commands and outputting results, for example.

**Example 2**

4 wines (marked 'A', 'B', 'C', 'D') were chosen from 2 different grapes. Wines 1 and 2 were of the same grape type but from different areas. Similarly wines 3 and 4 were of the same grape type but from different areas. The wines were divided into two sets. A training set containing 4 samples from each wine (16 samples in total). A test set containing 5 samples from each wine (20 samples in total). The training set was used in training the algorithm used in this test. And the test set was used in verifying the function of the array in discriminating the wines from each other. Parallel to this test a non-expert human panel consisting of 10 individuals was given 2 wines randomly from the test set and a training set (1 wine of each type). The humans had to recognize with all of their senses (smell, test, sight) the two wines by comparing them to given wines A, B, C and D.

The array according to the preferred embodiment of this invention consisted of 9 different selected non-specific binding spots and a protocol of the preferred embodiment of this invention was used. The wine samples for training and testing of the array were diluted in water. A single luminophore was used for detection and no extra references or normalizations were made.

The detection matrix (confusion matrix) for the method (left) and human panel (right) show the number of selected wines against the actual wine.

| Invention | | Selected | | | |
|-----------|---|---|---|---|---|
| | | A | B | C | D |
| Actual | A | 5 | | | |
| | B | | 5 | | |
| | C | | | 5 | |
| | C | | | | 5 |

| Humans | | Selected | | | |
|--------|---|---|---|---|---|
| | | A | B | C | D |
| Actual | A | 3 | 1 | 1 | |
| | B | | 1 | 1 | 3 |
| | C | | | 4 | 1 |
| | D | | 3 | | 2 |

The tested matrix showed good repeatability with up to 10 fold variance of signal between different wines and surfaces. Both the shape of the fingerprint and the absolute measured intensities can be used for identification and quantification purposes. Taken into account the experimental variance (<10% coefficient of variation) and intensity modulation created by different wines (10 fold). With this variance up to 5 different levels of binding for each array spot can be identified reliably. Thus the theoretical differentiation capability of this 9 spot array is $5^9$ = 1.9 million different samples.

**Example 3**

Figure 4 illustrates exemplary measurement plots of most significant, i.e. principal, components of bottled waters determined according to an advantageous embodiment of the invention, where 4 different bottled waters, 2 different tap waters and distilled water were compared by the method according to invention. The array according to the preferred embodiment of this invention consisted of 8 different selected non-specific binding spots and a protocol of the preferred embodiment of this invention was used. The water samples were used as such- A single luminophore was used for detection and no extra references or normalizations were made. Measurements were repeated six times for each water sample. The fingerprints were analysed by principal component analysis. The two most significant principal components (PC1 and PC2) are plotted in figure 2. Water samples are marked in the figure with letters a thru f. The distilled water is marked as cntrl. The overlapping water samples (c, d) are bottled waters that use the same raw water source but are marketed under different brand. Samples a) and e) were the tap waters from different raw water source (a=river water, e=underground well water).

**Claims**

1.  A method for fingerprinting a sample such as wine or water employing an array of at least two of different interacting surfaces, at least one of which comprises a non-specific interacting material non-specifically interacting with said sample, at least one labelling reactant, or combination of the sample and at least one labelling reactant, wherein said method comprises the following steps:

    a) introducing said sample and at least one labelling reactant to interact with said interacting surfaces of said array, wherein said labelling reactant is adapted to change at least one electromagnetically readable property of at least one interacting surface of said array;
    b) detecting at a predetermined time point said electromagnetically readable property of at least the non-specific interacting material of said at least two different interacting surfaces of said array to obtain a fingerprint of said sample; and
    c) determining said sample by comparing said fingerprint of said sample with

    i) at least one fingerprint of at least one corresponding sample,
    ii) at least one fingerprint of an array obtained without a sample, and/or
    iii) at least one fingerprint of known samples.

2.  The method according to claim 1, wherein the sample and/or at least one labelling reactant is introduced to a dilution medium before introducing to the interacting surfaces of said array, or introduced into a liquid medium before interacting with said interacting surfaces of the array.

3.  The method according to any of previous claims, wherein said interacting surfaces are washed with at least one washing medium before detecting said electromagnetically readable property of said interacting surfaces.

4.  The method according to any of previous claims, wherein at least one further labelling reactant is employed, said labelling reactant being adapted to interact in the conditions prevailing in the method specifically to at least one interacting surface of the different interacting surfaces of the array.

5.  The method according to any of previous claims, wherein:

    a) the sample is introduced to the interacting surfaces of said array before any labelling reactant, or
    b) at least one labelling reactant is introduced to the interacting surfaces of said array before the sample, or
    c) the sample and at least one labelling reactant is introduced to the interacting surfaces of said array together.

6.  Use of an array in a method of any of claims 1-5, wherein the array comprises at least two of different surface spots, the spots representing differently coated or modified surfaces, each forming a differently behaving non-specifically interacting surface.

7. The use according to claim 6, wherein the array comprises at least three interacting surfaces, where the two of which comprise similar type interacting surfaces.

8. The use according to claim 6 or 7, wherein at least 1, preferably at least 2, even more preferably at least 3 of the interacting surfaces are selected from each of at least 2, preferably at least 3, of the groups of interacting surfaces selected from the peptides, proteins, detergents, surfactants, carbohydrates and/or derivatives, and polymers.

9. The use according to any of claims 6-8, wherein the array comprises plurality of same type interacting surfaces, the interacting force, such as binding force of which is adapted to change gradually or continuously between the minimum and maximum interacting force values, and/or wherein the array comprises plurality of interacting surfaces, the type of which is adapted to change gradually or continuously from one type to another type.

10. The use according to any of claims 6-9, wherein the array comprises an additional surface essentially encapsulating said interacting surfaces.

11. The use according to any of claims 6-10, wherein essential portion, i.e. at least 10 %, preferably at least 30 %, even more preferably at least 50 % of the interacting surfaces are selected from groups of interacting surfaces selected from peptides, proteins, detergents, surfactants, carbohydrates and/or derivatives, and polymers, the polymer being optionally physically treated or chemically treated with an acid, base and/or solvent.

12. A computer program adapted to carry out the method of claim 1.

**Patentansprüche**

1. Verfahren zum Fingerprinting einer Probe, wie z. B. Wein oder Wasser, unter Verwendung einer Anordnung von wenigstens zwei verschiedenen wechselwirkenden Oberflächen, von denen wenigstens eine ein nichtspezifisch wechselwirkendes Material umfasst, das nichtspezifisch mit der Probe, wenigstens einem Markierungsreagens oder einer Kombination der Probe und wenigstens einem Markierungsreagens wechselwirkt, wobei das Verfahren folgende Schritte umfasst:

a) Einführen der Probe und wenigstens eines Markierungsreagens, um mit den wechselwirkenden Oberflächen der Anordnung zu wechselwirken, wobei das Markierungsreagens dafür ausgelegt ist, wenigstens eine elektromagnetisch auslesbare Eigenschaft wenigstens einer wechselwirkenden Oberfläche der Anordnung zu verändern;
b) zu einem vorbestimmten Zeitpunkt Erfassen der elektromagnetisch auslesbaren Eigenschaft wenigstens des nichtspezifisch wechselwirkenden Materials der wenigstens zwei verschieden wechselwirkenden Oberflächen der Anordnung, um einen Fingerprint der Probe zu erhalten; und
c) Bestimmen der Probe durch Vergleichen des Fingerprints der Probe mit

i) wenigstens einem Fingerprint wenigstens einer entsprechenden Probe,
ii) wenigstens einem Fingerprint einer ohne eine Probe erhaltenen Anordnung und/oder
iii) wenigstens einem Fingerprint bekannter Proben.

2. Verfahren gemäß Anspruch 1, wobei die Probe und/oder wenigstens ein Markierungsreagens in ein Verdünnungsmedium eingeführt wird, bevor es zu den wechselwirkenden Oberflächen der Anordnung eingeführt wird, oder in ein flüssiges Medium eingeführt wird, bevor es mit den wechselwirkenden Oberflächen der Anordnung wechselwirkt.

3. Verfahren gemäß einem der vorstehenden Ansprüche, wobei die wechselwirkenden Oberflächen mit wenigstens einem Waschmedium gewaschen werden, bevor die elektromagnetisch auslesbare Eigenschaft der wechselwirkenden Oberflächen erfasst wird.

4. Verfahren gemäß einem der vorstehenden Ansprüche, wobei wenigstens ein weiteres Markierungsreagens eingesetzt wird, wobei das Markierungsreagens dafür ausgelegt ist, unter den Bedingungen, die bei dem Verfahren herrschen, spezifisch mit wenigstens einer wechselwirkenden Oberfläche der verschiedenen wechselwirkenden Oberflächen der Anordnung zu wechselwirken.

**5.** Verfahren gemäß einem der vorstehenden Ansprüche, wobei:

a) die Probe zu den wechselwirkenden Oberflächen der Anordnung vor jeglichem Markierungsreagens eingeführt wird oder

b) wenigstens ein Markierungsreagens vor der Probe zu den wechselwirkenden Oberflächen der Anordnung eingeführt wird oder

c) die Probe und wenigstens ein Markierungsreagens gemeinsam zu den wechselwirkenden Oberflächen der Anordnung eingeführt werden.

**6.** Verwendung einer Anordnung bei einem Verfahren gemäß einem der Ansprüche 1-5, wobei die Anordnung wenigstens zwei verschiedene Oberflächenstellen umfasst, wobei die Stellen unterschiedlich beschichtete oder modifizierte Oberflächen darstellen, die jeweils eine sich unterschiedlich verhaltende nichtspezifisch wechselwirkende Oberfläche bilden.

**7.** Verwendung gemäß Anspruch 6, wobei die Anordnung wenigstens drei wechselwirkende Oberflächen umfasst, von denen zwei wechselwirkende Oberflächen von ähnlichem Typ umfassen.

**8.** Verwendung gemäß Anspruch 6 oder 7, wobei wenigstens 1, vorzugsweise wenigstens 2, noch bevorzugter wenigstens 3 der wechselwirkenden Oberflächen ausgewählt sind aus jeder von wenigstens 2, vorzugsweise wenigstens 3 der Gruppen von wechselwirkenden Oberflächen ausgewählt aus Peptiden, Proteinen, Detergenzien, grenzflächenaktiven Mitteln, Kohlenhydraten und/oder Derivaten und Polymeren.

**9.** Verwendung gemäß einem der Ansprüche 6-8, wobei die Anordnung eine Vielzahl von wechselwirkenden Oberflächen vom gleichen Typ umfasst, deren Wechselwirkungskraft, wie z. B. die Bindungskraft, dafür ausgelegt ist, dich allmählich oder kontinuierlich zwischen den Mindest- und Höchstwerten der Wechselwirkungskraft zu verändern, und/oder wobei die Anordnung eine Vielzahl von wechselwirkenden Oberflächen umfasst, deren Typ dafür ausgelegt ist, sich allmählich oder kontinuierlich von einem Typ zu einem anderen Typ zu verändern.

**10.** Verwendung gemäß einem der Ansprüche 6-9, wobei die Anordnung eine zusätzliche Oberfläche umfasst, die die wechselwirkenden Oberflächen im Wesentlichen einkapselt.

**11.** Verwendung gemäß einem der Ansprüche 6-10, wobei ein wesentlicher Teil, d. h. wenigstens 10 %, vorzugsweise wenigstens 30 %, noch bevorzugter wenigstens 50 %, der wechselwirkenden Oberflächen ausgewählt sind aus Gruppen von wechselwirkenden Oberflächen ausgewählt aus Peptiden, Proteinen, Detergenzien, grenzflächenaktiven Mitteln, Kohlenhydraten und/oder Derivaten und Polymeren, wobei das Polymer gegebenenfalls physikalisch behandelt oder mit einer Säure, Base und/oder einem Lösungsmittel chemisch behandelt ist.

**12.** Computerprogramm, das zum Ausführen des Verfahrens gemäß Anspruch 1 ausgelegt ist.

**Revendications**

**1.** Procédé de détermination d'empreinte digitale d'un échantillon tel que du vin ou de l'eau au moyen d'un réseau d'au moins deux de différentes surfaces d'interaction, dont au moins une comprend un matériau d'interaction non spécifique interagissant de façon non spécifique avec ledit échantillon, au moins un réactif de marquage, ou une combinaison de l'échantillon et d'au moins un réactif de marquage, ledit procédé comprenant les étapes suivantes :

a) introduction dudit échantillon et d'au moins un réactif de marquage pour interagir avec lesdites surfaces d'interaction dudit réseau, où ledit réactif de marquage est adapté pour modifier au moins une propriété mesurable de façon électromagnétique d'au moins une surface d'interaction dudit réseau ;
b) détection à un temps prédéterminé de ladite propriété mesurable de façon électromagnétique d'au moins le matériau d'interaction non spécifique desdites au moins deux surfaces d'interaction différentes dudit réseau pour obtenir une empreinte digitale dudit échantillon ; et
c) détermination dudit échantillon par comparaison de ladite empreinte digitale dudit échantillon à

i) au moins une empreinte digitale d'au moins un échantillon correspondant,
ii) au moins une empreinte digitale d'un réseau obtenu sans échantillon, et/ou
iii) au moins une empreinte digitale d'échantillons connus.

**2.** Procédé selon la revendication 1, dans lequel l'échantillon et/ou au moins un réactif de marquage est introduit dans un milieu de dilution avant l'introduction dans les surfaces d'interaction dudit réseau, ou introduit dans un milieu liquide avant interaction avec lesdites surfaces d'interaction du réseau.

**3.** Procédé selon l'une quelconque des revendications précédentes, dans lequel lesdites surfaces d'interaction sont lavées avec au moins un milieu de lavage avant la détection de ladite propriété mesurable de façon électromagnétique desdites surfaces d'interaction.

**4.** Procédé selon l'une quelconque des revendications précédentes, dans lequel au moins un réactif de marquage supplémentaire est utilisé, ledit réactif de marquage étant adapté pour interagir dans les conditions prévalentes dans le procédé spécifiquement avec au moins une surface d'interaction des différentes surfaces d'interaction du réseau.

**5.** Procédé selon l'une quelconque des revendications précédentes, dans lequel :

a) l'échantillon est introduit dans les surfaces d'interaction dudit réseau avant un réactif de marquage quelconque, ou
b) au moins un réactif de marquage est introduit dans les surfaces d'interaction dudit réseau avant l'échantillon, ou
c) l'échantillon et au moins un réactif de marquage est introduit dans les surfaces d'interaction dudit réseau conjointement.

**6.** Utilisation d'un réseau dans un procédé de l'une quelconque des revendications 1 à 5, dans laquelle le réseau comprend au moins deux de différent points de surface, les points représentant des surfaces revêtues ou modifiées de façon différente, chacune formant une surface d'interaction se comportant de façon non spécifiquement différente.

**7.** Utilisation selon la revendication 6, dans laquelle le réseau comprend au moins trois surfaces d'interaction, dont deux comprennent des surfaces d'interaction de type similaire.

**8.** Utilisation selon la revendication 6 ou 7, dans laquelle au moins 1, de préférence au moins 2, encore plus préférablement au moins 3 des surfaces d'interaction sont choisies parmi chacun d'au moins 2, de préférence au moins 3, des groupes de surfaces d'interaction choisis parmi les peptides, protéines, détergents, tensioactifs, glucides et/ou dérivés, et polymères.

**9.** Utilisation selon l'une quelconque des revendications 6 à 8, dans laquelle le réseau comprend une pluralité des surfaces d'interaction du même type, la force d'interaction, dont la force de liaison est adaptée pour changer progressivement ou de façon continue entre les valeurs de force d'interaction minimale et maximale, et/ou dans laquelle le réseau comprend une pluralité de surfaces d'interaction, dont le type est adapté pour changer progressivement ou de façon continue d'un type à un autre type.

**10.** Utilisation selon l'une quelconque des revendications 6 à 9, dans laquelle le réseau comprend une surface additionnelle encapsulant essentiellement lesdites surfaces d'interaction.

**11.** Utilisation selon l'une quelconque des revendications 6 à 10, dans laquelle la partie essentielle, à savoir au moins 10 %, de préférence au moins 30 %, encore plus préférablement au moins 50 % des surfaces d'interaction sont choisis parmi des groupes de surfaces d'interaction choisies parmi des peptides, des protéines, des détergents, des tensioactifs, des glucides et/ou dérivés, et des polymères, le polymère étant facultativement physiquement traité ou chimiquement traité avec un acide, une base et/ou un solvant.

**12.** Programme informatique adapté pour conduire le procédé de la revendication 1.

**FIG. 1**

**FIG. 2A**

**FIG. 2B**

**FIG. 2C**

FIG. 2D

FIG. 2E

FIG. 2F

**FIG. 3A**

**FIG. 4**

## REFERENCES CITED IN THE DESCRIPTION

**Patent documents cited in the description**

- WO 2001055702 A
- US 5891738 A
- WO 2003042698 A
- WO 2004048937 A
- WO 2007077291 A
- US 2009017477 A
- WO 0016101 A

**Non-patent literature cited in the description**

- *Journal of Biomedicine and Biotechnology,* 2003, vol. 5, 257-266
- **YURI VLASOV.** IUPAC Technical report. *Pure Appl.Chem.,* 2005, vol. 77 (11), 1965-1983
- **YOU, MIRANDA.** *Nature Nanotechnology,* 2007, vol. 2, 318-323
- **NAREOJA et al.** *Journal of Immunological Methods,* vol. 345 (1-2), 80-89
- **BARTZ A.E.** Basic Statistical Concepts. Prentice Hall, 1999